# EUROPÄISCHE PATENTSCHRIFT

(11) **EP 0 573 746 B1**
(45) Veröffentlichungstag und Bekanntmachung des Hinweises auf die Patenterteilung: **20.12.1995**
(21) Anmeldenummer: 93104831.8
(22) Anmeldetag: 24.03.1993
(51) Int. Cl.: A61B 1/00

(54) **Endoskop mit einem flexiblen Schaft**
Endoscope with a flexible shaft
Endoscope à tige flexible

(30) Priorität: 06.06.1992 DE 4218706
(43) Veröffentlichungstag der Anmeldung: 15.12.1993
(73) Patentinhaber: Richard Wolf GmbH, 75438 Knittlingen (DE)
(72) Erfinder: Heimberger, Rudolf, W-7519 Oberderdingen (DE)
(74) Vertreter: Wilcken, Hugo

(56) Entgegenhaltungen:
- EP-A- 0 306 723
- WO-A-85/02101
- DE-A- 241 886
- US-A- 4 911 148

## Beschreibung

Die Erfindung geht aus von einem Endoskop mit einem flexiblen Schaft, dessen proximales Ende in ein Anschlußteil übergeht und dessen distales Ende durch Verstellung eines Betätigungsdrahtes in verschiedene Positionen lenkbar ist, und mit einem Handhabeteil, an dessen distalem Ende das Anschlußteil lösbar ankuppelbar ist und an dessen proximalem Ende ein Okularteil angeordnet ist, wobei im Handhabeteil eine von Hand zu betätigende Steuerung für den Betätigungsdraht vorgesehen ist.

Es ist bekannt, daß dünne flexible Endoskope mit einem Durchmesser, der kleiner als 2,5 mm ist, aufgrund ihrer filigranen Struktur anfällig für Beschädigungen sind. Neben den möglichen Beschädigungen kommt es auch häufig vor, daß nach mehrmaligem Gebrauch Bildleiter oder Lichtleiter brechen, wodurch das flexible Endoskop unbrauchbar wird. Es handelt sich hierbei um flexible Endoskope, bei denen innerhalb des angegebenen sehr geringen Durchmessers einerseits Lichtleiter für die ausreichende Ausleuchtung des Untersuchungs- und Operationsgebietes und Bildleiter mit genügend Bildpunkten, um im Behandlungsgebiet auch Manipulationen mit Hilfsinstrumenten, wie z.B. an Körpergewebe, ohne Gefährdung des Patienten vornehmen zu können, angeordnet werden müssen. Andererseits muß mindestens ein Kanal für Hilfsinstrumente oder zum Zu- und Abführen von Fluiden vorgesehen werden, und das Instrument muß vom Anwender über ein ebenfalls innerhalb des Instrumentes angeordnetes Steuersystem , z.B. einen bis zum distalen Ende führenden Betätigungsdraht, verfügen, um den anatomischen Gegebenheiten der Körperhöhle oder des Körperkanales, wie beispielsweise Blutgefäße oder Harnleiter, entsprechend führbar zu sein. Weiterhin ist ein Steuersystem bzw. eine Steuerung notwendig, um verschiedene Stellen des Behandlungsgebietes mit dem distalen Ende des Endoskopschaftes erreichen zu können.

Ist bei herkömmlichen flexiblen Endoskopen beispielsweise ein Lichtleiter oder Bildleiter gebrochen, so ist eine umfangreiche Reparatur erforderlich. Zum Teil kann sie sogar aufgrund der Bauweise des Endoskopes gar nicht erst durchgeführt werden. Deshalb ist man dazu übergegangen, ein solches komplettes Instrument in einzelne Baugruppen zu gliedern, die dann bei Beschädigungen bis zur Reparatur auf einfache Weise gegen funktionierende Baugruppen ersetzt werden können.

Es sind Instrumente bekannt, bei denen die Handhabe und das Okular nicht als bauliche Einheit, sondern als aneinander festlegbare Baugruppen ausgebildet sind. Ein Beispiel hierfür entnimmt man der WO 85/02101, die einen nicht steuerbaren Katheter betrifft und für die nachfolgend näher beschriebene Erfindung ausschließlich als Beispiel für den Aufbau eines gattungsgemäßen Instrumentes aus einzelnen Baugruppen genannt ist.

Bei flexiblen Endoskopen ist es üblich, den flexiblen Schaft mit proximalem Anschlußteil als eine Einheit auszubilden, die an einer mit einem Okular fest verbundenen Handhabe lösbar festgelegt werden kann. Ein Beispiel für eine derartige Ausführungsform zeigt das US-Patent 4 911 148. Bei diesem Endoskop wird eine Baugruppe durch den flexiblen, mit Lichtleiter, Bildleiter, Instrumentenkanal und Betätigungsdraht versehenen Schaft mit Anschlußteil gebildet. Die zweite Baugruppe stellt eine Handhabe mit daran festgelegtem, fokussierbarem Okular dar. Der Bildleiter ragt bei dieser Ausführungsform proximal über das Ende der ersten Baugruppe hinaus und ist von einer Hülse umgeben, welche mit einer zweiten Hülse im Handhabeteil beim Zusammenbau des Endoskopes korrespondiert. Hierdurch wird eine Zentrierung der beiden Baugruppen gewährleistet.

Der Nachteil einer derartigen Ausführungsform liegt in einer sehr aufwendigen Mechanik für die Verstellung des Betätigungsdrahtes, was zugleich auch eine mit großem Aufwand verbundene Herstellung bedeutet. Liegt beispielsweise eine Beschädigung des Okularteiles vor, so wirkt es sich nachteilig aus, daß das Instrument aus nur zwei Baugruppen besteht, denn es muß die gesamte Handhabe - Okular - Baugruppe ausgetauscht und repariert oder gar weggeworfen werden, da das Okularteil nicht als einzelnes Bauelement vom Anwender abnehmbar ist. Desweiteren ist es für den Anwender sehr ungewohnt, die Steuerung des Endoskopschaftes durch Drehen eines Verstellringes vornehmen zu müssen, so daß sich für den Bediener eine unbequeme Handhabung des Instrumentes ergibt.

Durch die Erfindung soll ein demgegenüber einfacheres und zudem kostengünstigeres flexibles Endoskop vorgeschlagen werden, das im übrigen alle auch schon vorher genannten Anforderungen erfüllt. Insbesondere soll ein flexibles, aus Baugruppen aufgebautes Endoskop angegeben werden, bei dem die einzelnen Baugruppen leicht miteinander verbunden und leicht voneinander gelöst werden können, wobei die Steuerung des flexiblen Schaftes durch eine einfache Mechanik erfolgt und eine bessere Handhabung durch da Bediener gewährleistet werden soll.

Diese Aufgabe wird erfindungsgemäß dadurch gelöst, daß bei dem einleitend erwähnten Endoskop mit einem flexiblen Schaft das proximale Ende des Betätigungsdrahtes mit einer Aufnahme in der Steuerung verbunden ist und daß die Aufnahme durch Betätigung eines Verstellhebels zwecks Freigabe des erwähnten Drahtendes zu öffnen ist.

Die Mechanik der Steuerung ist beispielsweise so konstruiert, daß die einzelnen Teile der Steuerung derart zusammenwirken, daß ausgehend von der Neutralstellung des zum Steuersystem gehörenden Verstellhebels ein Formschluß zwischen dem proximalen Ende des Betätigungsdrahtes und einer Gewindehalbschale bei einer Bewegung in distaler Richtung des Verstellhebels dann aufgehoben ist, wenn die Federkraft einer zur Steuerung gehörenden Feder überwunden worden ist, wobei ein sich innerhalb der Steuerung befindender Anschlagstift auf einem Schenkel des Haltewinkels derart gleitet, daß die Feder zusammengedruckt wird. Vorteilhaft an dieser Lösung ist, daß bei Beschädigung einer Baugruppe des Instrumentes diese einfach entfernt und repariert oder ersetzt werden kann.

Die eigentliche Steuerung des Endoskopes, d.h. das Ablenken des flexiblen distalen Endes in verschiedene Positionen, erfolgt mit der gleichen Steuerung. Nur wird hierfür der zur Steuerung gehörende Verstellhebel ausgehend von der Neutralstellung in eine andere Richtung, also nicht in distale Richtung sondern zum Beispiel in die entgegengesetzte, proximale Richtung, bewegt. Der Formschluß zwischen der Gewindehalbschale der Steuerung und dem Betätigungsdrahtende bleibt hierbei erhalten. Somit ist zusätzlich eine als vom Bediener vorteilhaft bzw. angenehm empfundene Handhabung des flexiblen Endoskopes mittels des erfindungsgemäßen Steuerungssystemes gewährleistet, welche zudem einfach und sicher funktioniert.

Desweiteren ist der Betätigungsdraht distalseitig einer dicker ausgebildeten Drahtstrecke durch eine daran festgelegte Druckfeder gegenüber einem Anschlag des Anschlußteiles beweglich. Werden nun das Anschlußteil und das Handhabeteil versehentlich zusammengesteckt, obwohl sich der Verstellhebel nicht in der Offenstellung befindet was dann der Fall ist, wenn der Verstellhebel nicht in distaler Richtung, also nach unten bewegt wurde, so wird die Druckfeder zusammengedrückt und verhindert somit eine Stauchung des Betätigungsdrahtes und eine Beschädigung eines auf diesem befindlichen Gewindeabschnittes.

Eine vorteilhafte Weiterbildung des erfindungsgemäß ausgebildeten Endoskopes liegt darin, daß gleichzeitig eine mögliche Überlastung der Steuerung und des distalen Endoskopendes vermieden werden kann. Der Überlastschutz ist dadurch gegeben, daß bei zu starker Belastung des Betätigungsdrahtes die formschlüssige Verbindung zwischen dem Gewindeabschnitt und der Gewindehalbschale entgegen dem Federdruck der Feder innerhalb des Steuersystems aufgehoben wird, so daß sich ein kombinierter Formschluß - Kraftschluß ergibt.

Weiterhin ist das erfindungsgemäße Instrument so ausgebildet, daß der Bildleiter des flexiblen Endoskopes ab etwa der Mitte des Anschlußteiles in einem dünnen Rohr, das als Schutz- und Führungsrohr für den Bildleiter dient, geführt ist, welches zusammen mit dem Bildleiter proximalwärts das Anschlußteil so weit überragt, daß bei komplettiertem Endoskop der Bildleiter und das Rohr direkt an einem distalen Verschlußplättchen des Okularteiles enden. Auch die Handhabe ist mit einem Führungsrohr ausgestattet, welches zur Aufnahme des Bildleiters zusammen mit dem ihn umgehenden Rohr ausgebildet ist und unmittelbar vor einem Anschlag der Handhabe endet. Das Verschlußplättchen des Okularteiles liegt bei zusammengebauten Bauteilen an der proximalen Seite des Anschlages an. Die Vorteile einer solchen Ausführungsform sind die, daß einerseits ein Schutz für den Bildleiter gewährleistet ist und daß andererseits Führungsrohre vorhanden sind, die eine einfache Zentrierung bzw. Fixierung der einzelnen Bauelemente beim Montieren ermöglichen.

Entsprechend ist auch der Betätigungsdraht ab etwa der Mitte des Anschlußteiles in einem Führungsrohr geführt, welches proximal an dem Steuerteil des Handhabeteiles festgelegt und so lang ist, daß bei komplettiertem Endoskop der Betätigungsdraht mit dem Steuerteil der Handhabe sicher zusammenwirken kann.

Um im übrigen eine leicht herstellbare, leicht lösbare und die korrekte Lage der Einzelelemente gewährleistende Verbindung zwischen dem Anschlußteil und dem Handhabeteil zu ermöglichen, ist das Anschlußteil mit einer Längsnut versehen, welche mit einem Positionierstift des Handhabeteiles, beispielsweise als Kugelraste ausgebildet, korrespondiert.

Zweckmäßigerweise ist am distalen Ende des Handhabeteiles ein Runddraht angeordnet, welcher beim Montieren des Handhabeteiles und des Anschlußteiles in eine Ringnut des Anschlußteiles einrastet. Damit der Runddraht verformbar ist und mit ausreichendem Spiel in der Ringnut sitzt, sollte er nicht vollständig zu einem Ring geschlossen sein. Außerdem können dann das Anschlußteil und das Handhabeteil leichter wieder voneinander gelöst werden.

Schließlich kann eine dichte Verbindung des Handhabeteiles und des Anschlußteiles dadurch gewährleistet werden, daß am distalen Ende des Handhabeteiles ein O - Ring zwischen dem Handhabeteil und dem Anschlußteil angeordnet wird.

Die Erfindung wird nachfolgend anhand eines in der anliegenden Zeichnung dargestellten Ausführungsbeispieles näher erläutert. Es zeigen:
- Figur 1: ein komplettes erfindungsgemäßes flexibles Endoskop im teilweisen Schnitt,
- Figur 2: einen vergrößert dargestellten Ausschnitt des proximalen Endes des Anschlußteiles in Verbindung mit dem distalen Ende des Handhabeteiles,
- Figur 3: einen Schnitt durch das erfindungsgemäß ausgebildete Handhabeteil mit Steuerung,
- Figur 4: einen Querschnitt des distalen flexiblen Endoskopteiles entsprechend der Schnittlinie IV - IV nach Figur 1,
- Figur 5: das Anschlußteil des flexiblen Endoskopes in teilweisem Schnitt,
- Figur 6: eine ungeschnittene Darstellung des Anschlußteiles nach Figur 5 in hierzu um 90° gedrehter Lage,
- Figur 7: einen Schnitt entlang der Linie VII - VII gemäß Figur 6 und
- Figuren 8a und 8b: Schnitte in vergrößertem Maßstab durch das proximale Ende des Handhabeteiles mit der Steuerung in zwei verschiedenen Stellungen.

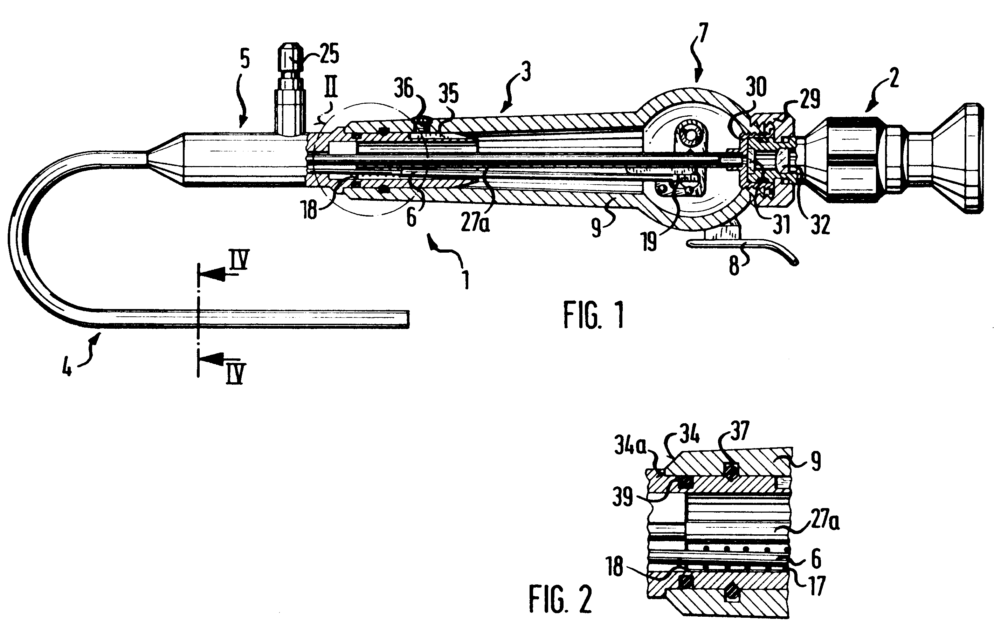

Das flexible Endoskop 1 besteht gemäß Figur 1 im wesentlichen aus einem fokussierbaren Okularteil 2, einem Handhabeteil 3 und einem flexiblen Schaft 4 mit proximalem, in das Handhabeteil 3 passendem Anschlußteil 5. Die einzelnen Baugruppen 2,3 und 4,5 sind, wie später noch näher beschrieben wird, lösbar miteinander verbunden. Innerhalb des Handhabeteiles 3 ist die Steuerung 7 vorgesehen.

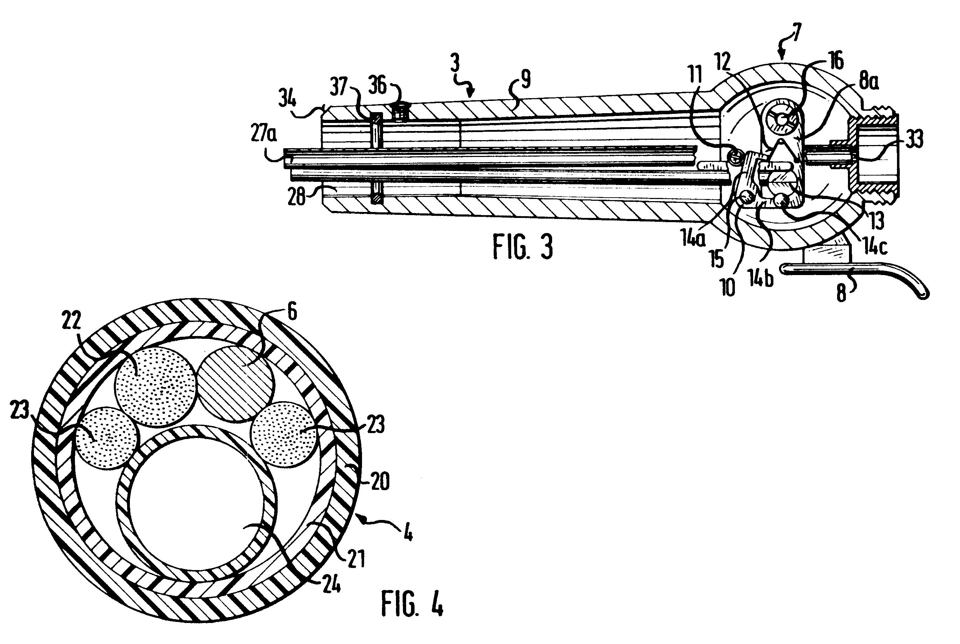

Die Steuerung 7, welche sich gemäß Figur 3 in der Neutralstellung befindet, besteht im wesentlichen aus einem außerhalb des Handhabegehäuses 9 befindlichen Verstellhebel 8, einem Haltewinkel 10, einem Anschlagstift 11, einer Feder 12 und einer mit dem innenliegenden Teil 8a des Verstellhebels 8 fest verbundenen Gewindehalbschale 13. Der Verstellhebel 8 und sein innenliegender Teil 8a sind starr miteinander über eine Lagerachse 16 verbunden, während sich der Haltewinkel 10 um eine Lagerachse 15 gegen die Kraft der Feder 12 verschwenken läßt.

Die genaue Funktion der Mechanik im Zusammenwirken mit dem für die Steuerung 7 erforderlichen Betätigungsdraht 6 wird später beschrieben. Zunächst sollen die einzelnen Baugruppen und ihre Verbindungen zu den benachbarten Baugruppen näher erläutert werden.

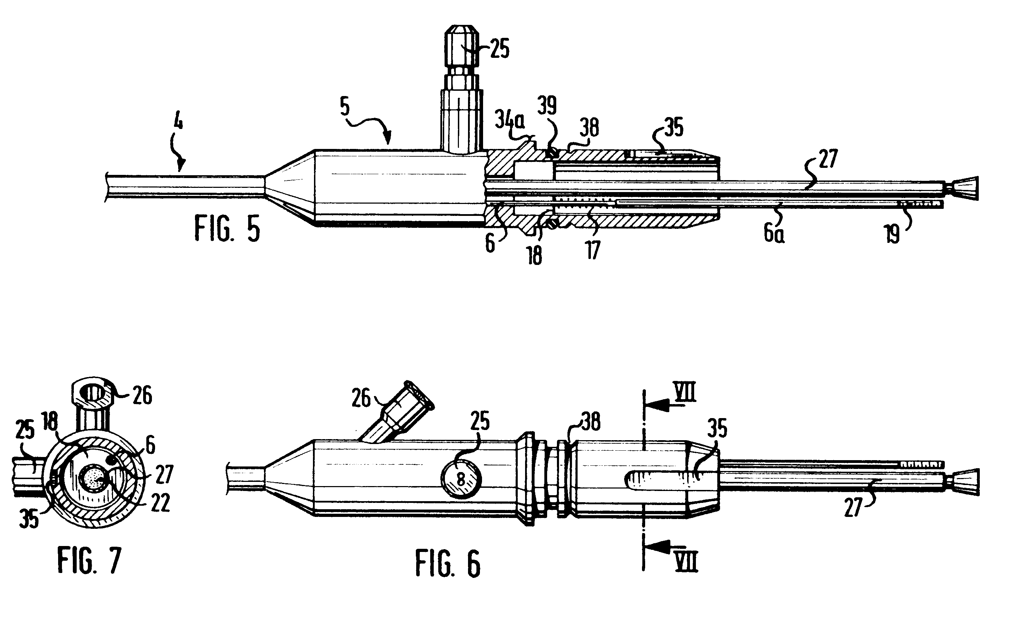

Der Betätigungsdraht 6 ist, wie insbesondere aus Figur 5 hervorgeht, distalseitig eines dicker ausgebildeten Drahtteiles 6a durch eine daran festgelegte Druckfeder 17 gegenüber einem Anschlag 18 des Anschlußteiles 5 beweglich gelagert. Figur 2 stellt diesen Abschnitt in vergrößertem Maßstab heraus. Zusätzlich weist der Betätigungsdraht 6 am proximalen Ende noch einen mit Gewinde versehenen Abschnitt 19 auf. Werden die Bauteile 3 und 5 nun versehentlich zusammengesteckt, obwohl sich der Verstellhebel 8 bzw. die Aufnahme für den Drahtabschnitt 19 nicht in der Offenstellung befindet, so wird die Feder 17 zusammengedrückt und wirkt somit einer Stauchung des Betätigungsdrahtes 6 und einer Beschädigung des Abschnittes 19 entgegen.

In Figur 1 sind die einzelnen benachbarten Baugruppen des komplett dargestellten flexiblen Endoskopes 1 erkennbar, dessen fokussierbares Okularteil 2, das in üblicher Weise aufgebaut ist und daher nicht weiter beschrieben wird, distalseitig mit einer Überwurfmutter 29 versehen ist. Diese greift in ein entsprechend ausgebildetes Gewinde 30 des proximalen Endes des Handhabeteiles 3 ein. Weiter ist das Okularteil 2 distalseitig durch ein Verschlußplättchen 31 aus lichtdurchlässigem und kratzfestem Material, wie z.B Saphir, hermetisch verschlossen. Beim Befestigen des Okularteiles 2 am Handhabeteil 3 gewährleistet eine Dichtung 32, beispielsweise als O - Ring ausgebildet, im Zusammenspiel mit der Überwurfmutter 29 und dem passenden Gegengewinde 30 des Handhabeteiles 3 eine feste, luft- und flüssigkeitsdichte, lösbare Verbindung der beiden Endoskopbaugruppen 2 und 3.

Der flexible Schaft 4 besteht, wie insbesondere aus Figur 4 hervorgeht, aus einem Innenschaft 21, welcher von einem Hüllschlauch 20 umgeben ist. Der Innenschaft 21 kann mit einer oder mehreren in Kunststoff eingebetteten Drahtwendeln verstärktsein.

Der Innenschaft 21 umfaßt einen Bildleiter bzw. ein Bildleiterbündel 22 sowie Lichtleiter 23. Daneben sind noch der bereits erwähnte Betätigungsdraht 6 für die Steuerung sowie ein Kanal 24 für Hilfsinstrumente vorgesehen. Im übrigen können die Elemente 22 bis 24 im flexiblen Schaft 4 in ebenfalls bekannter Weise z. B. mit einem Kunststoff vergossen sein.

Die Figuren 5 und 6 stellen das starre Anschlußteil 5 dar, in welches der flexible Schaft 4 übergeht. An dem Anschlußteil 5 ist ein Anschluß 25 für ein Lichtleitkabel vorgesehen. In diesem Anschluß münden die Lichtleiter 23. Zusätzlich ist ein seitlich abgewinkelter Hilfsinstrumenteneinführstutzen 26 vorhanden, in welchen der Kanal 24 für die Hilfsinstrumente mündet.

Der oder die Bildleiter 22 sind ab etwa der Mitte des Anschlußteiles 5 in einem dünnen Rohr 27 geführt und überragen proximalwärts mit diesem zusammen das Anschlußteil 5 so weit, daß bei komplettiertem Endoskop 1 nach Figur 1 der Bildleiter 22 und das ihn umgebende Rohr 27, welches zum Schutz und gleichzeitig als Führung dient, direkt am distalen Verschlußplättchen 31 des Okularteiles 2 enden. In gleicher Weise wird ebenfalls etwa ab der Mitte des Anschlußteiles 5 der Betätigungsdraht 6 in einem Führungsrohr 28 geführt, das proximal im Handhabeteil 3 festgelegt und so lang ist, daß bei komplettiertem Endoskop 1 der Betätigungsdraht 6 mit der Steuerung 7 des Handhabeteiles 3 sicher zusammenwirken kann.

Das Handhabeteil 3 weist außerdem noch ein Führungsrohr 27a auf, welches zur Aufnahme des Bildleiters 22 mit Rohr 27 ausgebildet ist und unmittelbar vor einem Anschlag 33 des Handhabeteiles 3 endet, an dessen proximaler Seite bei zusammengefügten Bauteilen 2 bis 5 das distale Verschlußplättchen 31 des Okularteiles 2 anliegt.

Um eine leicht herstellbare, leicht lösbare, luft- und feuchtigkeitsdichte und die korrekte Lage der Einzelelemente gewährleistende Verbindung zwischen dem Anschlußteil 5 und dem Handhabeteil 3 zu ermöglichen, hat das Anschlußteil 5 eine Längsnut 35, welche mit einem z.B. als Kugelraste ausgebildeten Positionierstift 36 des Handhabeteiles 3 korrespondiert. Desweiteren kann beim Zusammenbau der Teile 3 und 5 ein Runddraht 37, am distalen Ende des Handhabeteiles 3 angeordnet, in eine Ringnut 38 des Anschlußteiles 5 einrasten. Damit die Teile 3 und 5 wieder leicht durch Ziehen voneinander gelöst werden können, sollte der Runddraht 37 verformbar sein und mit ausreichendem Spiel in der Ringnut 38 sitzen. Dieses wird durch Wahl eines nicht vollständig zu einem Ring geschlossenen Runddrahtes 37 erreicht. Die Dichtheit der Verbindung dieser beiden Teile kann durch einen O - Ring 39 gewährleistet werden.

Das Zusammenwirken der erfindungsgemäß ausgebildeten Endoskopbauteile wird nachstehend näher erläutert.

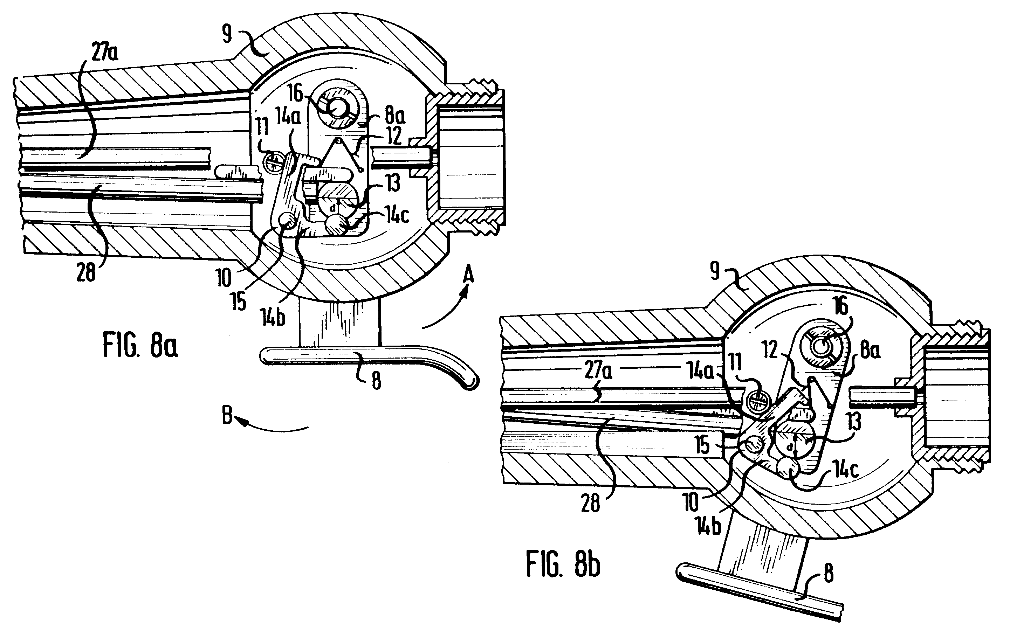

Soll das Anschlußteil 5 mit dem Handhabeteil 3 verbunden werden, so ist lediglich zu beachten, daß das Rohr 27 zum Schutz der Bildleiterbündel 22 und der Betätigungsdraht 6 mit den entsprechenden Führungsrohren 27a und 28 des Handhabeteiles 3 ausgerichtet sind, was dann erfüllt ist, wenn der Positionierstift 36 in die Längsnut 35 greift. Es wird nun von Hand der Verstellhebel 8 gemäß Figur 8a in Richtung B bewegt, und folglich vergrößert sich der Abstand d zwischen der Gewindehalbschale 13 und einem Gegendruckstück 14c, wie es Figur 8b zeigt. Es kann nun das proximale Ende 19 des Betätigungsdrahtes zwischen diese die Aufnahme für das proximale Drahtende 19 bildenden Teile 13 und 14c geschoben und das Anschlußteil 5 mit dem Handhabeteil 3 zusammengesteckt werden. Nach dem Zurückbewegen des Verstellhebels 8 in die Neutralstellung ist der Formschluß zwischen dem Drahtende 19 und der Aufnahme 13, 14c unter Einwirkung der Feder 12 hergestellt, da das freie Ende 14c des Hebelschenkels 14b das Drahtende 19 von unten in die Gewindehalbschale 13 drückt. Das distale Ende 34 des Handhabegehäuses 9 liegt dann an einer Schulter 34a des Anschlußteiles 5 an, während das Ankuppeln der beiden Teile 3 und 5 durch Eingreifen des Runddrahtes 37 in die Ringnut 38 erfolgt.

Analog zu diesem Vorgang erfolgt auch das Abkuppeln des Betätigungsdrahtes 6. Ausgehend von der in Figur 8a dargestellten Neutralstellung wird der Verstellhebel 8 in Richtung distales Instrumentenende, also die in Figur 8a in Richtung B gekennzeichnete Bewegung, gedrückt. Es ist nun die Position erreicht, wie sie Figur 8b zeigt. Bei dieser Stellung ist der Formschluß zwischen dem Betätigungsdraht 6 und der Gewindehalbschale 13 aufgehoben. Es mußte dabei die Federkraft der Feder 12 überwunden werden. Der ortsfeste Anschlagstift 11 wirkt dabei auf den Schenkel 14a des Haltewinkels 10 in der Weise ein, daß die Feder 12 zusammengedruckt wird. Dadurch wird der Haltewinkel 10 im Uhrzeigersinn um die Lagerachse 15 verschwenkt, wodurch der Schenkel 14b den Draht 6 freigibt. Man erkennt in Figur 8b, daß der Abstand d zwischen der Gewindehalbschale 13 und dem Schenkel 14b im Vergleich zu der in Figur 8a dargestellten Neutralstellung größer geworden ist. Es können nun das Handhabeteil 3 und das Anschlußteil 5 einfach durch Überwindung der Haltekraft zwischen dem Runddraht 37 und der Ringnut 38 auseinandergezogen werden.

Im Gegensatz zu Figur 8b besteht in der Darstellung nach Figur 8a Formschluß zwischen der Gewindehalbschale 13 der Steuerung 7 und dem Ende 19 des Betätigungsdrahtes 6, und der Schaft 4 kann durch Betätigen des Verstellhebels 8 in proximale Richtung und zurück, d.h. durch Bewegung des Verstellhebels 8 nach oben bzw. in die in Figur 8a mit A gekennzeichnete Richtung und zurück in üblicher Weise gesteuert werden, wobei der Betätigungsdraht 6 in Längsrichtung bewegt wird.

Die Steuerung 7 stellt gleichzeitig eine Überlastsicherung dar, denn wird der Betätigungsdraht 6 zu stark belastet, so öffnet sich die formschlüssige Verbindung zwischen dem Gewindeabschnitt 19 und der Gewindehalbschale 13 entgegen dem Federdruck der Feder 12.

## Patentansprüche

1. Endoskop (1) mit einem flexiblen Schaft (4), dessen proximales Ende in ein Anschlußteil (5) übergeht und dessen distales Ende durch Verstellung eines Betätigungsdrahtes (6) in verschiedene Positionen lenkbar ist, und mit einem Handhabeteil (3), an dessen distalem Ende das Anschlußteil (5) lösbar ankuppelbar ist und an dessen proximalem Ende ein Okularteil (2) angeordnet ist, wobei im Handhabeteil (3) eine von Hand zu betätigende Steuerung (7) für den Betätigungsdraht (6) vorgesehen ist, dadurch gekennzeichnet, daß das proximale Ende (19) des Betätigungsdrahtes (6) mit einer Aufnahme (13,14c) in der Steuerung (7) verbunden ist und daß die Aufnahme (13,14c) durch Betätigung eines Verstellhebels (8) zwecks Freigabe des erwähnten Drahtendes (19) zu öffnen ist.

2. Endoskop nach Anspruch 1, dadurch gekennzeichnet, daß der Verstellhebel (8), ausgehend von einer Neutralstellung, in einer Richtung zum Öffnen der Aufnahme (13,14c) bewegbar ist und daß die Aufnahme (13,14c) bei der Neutralstellung und beim Verstellen des Betätigungsdrahtes (6) geschlossen und formschlüssig mit dem proximalen Drahtende (19) verbunden ist.

3. Endoskop nach einem der Ansprüche 1 und 2, dadurch gekennzeichnet, daß der Verstellhebel (8) und ein innenliegender Hebelteil (8a) starr miteinander verbunden sind, und daß ein Haltewinkel (10) um eine Lagerachse (15) gegen die Kraft einer zur Steuerung (7) gehörenden Feder (12) verschwenkbar ist.

4. Endoskop nach einem der Ansprüche 1 bis 3, dadurch gekennzeichnet, daß, ausgehend von der Neutralstellung, bei einer Bewegung des Verstellhebels (8) in distaler Richtung, ein sich innerhalb der Steuerung (7) befindender Anschlagstift (11) auf einen Schenkel (14a) des Haltewinkels (10) wirkt, derart, daß die zur Steuerung gehörende Feder (12) zusammengedrückt und die Aufnahme (13,14c) geöffnet wird.

5. Endoskop nach einem der Ansprüche 1 bis 4, dadurch gekennzeichnet, daß der Betätigungsdraht (6) distalseitig eines dicker ausgebildeten Drahtabschnittes (6a) durch eine Druckfeder (17) gegenüber einem Anschlag (18) des Anschlußteiles (5) längsbeweglich abgestützt ist.

6. Endoskop nach Anspruch 5, dadurch gekennzeichnet, daß der Betätigungsdraht (6) proximal einen mit Gewinde versehenen Abschnitt (19) aufweist, der unter Einwirkung einer Federkraft mit einer Gewindehalbschale (13) in formschlüssigen Eingriff bringbar ist.

7. Endoskop nach Anspruch 6, dadurch gekennzeichnet, daß vor Überlastung des Betätigungsdrahtes (6) die formschlüssige Verbindung zwischen Gewindeabschnitt (19) und Gewindehalbschale (13) entgegen dem Federdruck der zur Steuerung gehörenden Feder (12) lösbar ist.

8. Endoskop nach einem der Ansprüche 1 bis 7, dadurch gekennzeichnet, daß der Bildleiter (22) des Endoskopes (1) ab etwa der Mitte des Anschlußteiles (5) in einem Rohr (27) geführt ist.

9. Endoskop nach einem der Ansprüche 1 bis 8, dadurch gekennzeichnet, daß das Handhabeteil (3) ein Führungsrohr (27a) aufweist, welches zur Aufnahme des Bildleiters (22) zusammen mit dessen Rohr (27) ausgebildet ist und unmittelbar vor einem Anschlag (33) des Handhabeteiles (3) endet.

10. Endoskop nach Anspruch 9, dadurch gekennzeichnet, daß ein distales Verschlußplättchen (31) des Okularteiles (2) an der proximalen Seite des Anschlages (33) anliegt.

11. Endoskop nach einem der Ansprüche 1 bis 10, dadurch gekennzeichnet, daß der Betätigungsdraht (6) in einem Führungsrohr (28) geführt ist, welches proximal im Handhabeteil (3) festlegbar ist.

12. Endoskop nach einem der Ansprüche 1 bis 11, dadurch gekennzeichnet, daß das Anschlußteil (5) eine Längsnut (35) aufweist, welche mit einem Positionierstift (36) des Handhabeteiles (3) korrespondiert.

13. Endoskop nach einem der Ansprüche 1 bis 12, dadurch gekennzeichnet, daß am distalen Ende des Handhabeteiles (3) ein Runddraht (37) angeordnet ist, der in eine Ringnut (38) des Anschlußteiles (5) einrasten kann.

## Claims

1. Endoscope (1) having a flexible shaft (4), the proximal end of which merges into a connection part (5) and the distal end of which can be steered into different positions by adjusting an actuation wire (6), and having a handle part (3) to the distal end of which the connection part (5) can be releasably coupled, and on the proximal end of which an ocular part (2) is arranged, wherein a control (7) to be actuated by hand for actuation wire (6) is provided in handle part (3), characterised in that the proximal end (19) of actuation wire (6) is connected to a receiver (13, 14c) in control (7), and in that receiver (13, 14c) can be opened by actuating an adjusting fever (8) for the purpose of releasing the said end (19) of the wire.

2. Endoscope according to claim 1, characterised in that the adjusting lever (8) can be moved into a direction for opening the receiver (13, 14c), starting from a neutral position, and in that in the neutral position and when adjusting actuation wire (6), receiver (13, 14c) is closed and connected positively to the proximal end (19) of the wire.

3. Endoscope according to one of claims 1 and 2, characterised in that adjusting lever (8) and an internal lever part (8a) are connected rigidly to one another, and in that a retaining angle (10) can be pivoted about a bearing axis (15) against the force of a spring (12) belonging to control (7).

4. Endoscope according to one of claims 1 to 3, characterised in that, starting from the neutral position, when moving adjusting lever (8) in the distal direction, a stop pin (11) situated within control (7) acts on a limb (14a) of the retaining angle (10) such that the spring (12) belonging to the control is compressed and receiver (13, 14c) is opened.

5. Endoscope according to one of claims 1 to 4, characterised in that actuation wire (6) is supported to be longitudinally movable on the distal side of a wire section (6a) designed to be thicker by means of a compression spring (17) opposite a stop (18) on connection part (5).

6. Endoscope according to claim 5, characterised in that proximally actuation wire (6) has a section (19) provided with a thread, which section (19) can be positively engaged with a thread half-shell (13) under the action of spring force.

7. Endoscope according to claim 6, characterised in that before overloading actuation wire (6) the positive connection between thread section (19) and thread half-shell (13) can be released counter to the spring pressure of spring (12) belonging to the control.

8. Endoscope according to one of claims 1 to 7, characterised in that image conductor (22) of endoscope (1) is guided in a tube (27) from about the centre of connection part (5).

9. Endoscope according to one of claims 1 to 8, characterised in that handle part (3) has a guide tube (27a) which is designed to receive image conductor (22) together with its tube (27) and terminates immediately before a stop (33) on handle part (3).

10. Endoscope according to claim 9, characterised in that a small distal closure plate (31) of ocular part (2) rests against the proximal side of stop (33).

11. Endoscope according to one of claims 1 to 10, characterised in that actuation wire (6) is guided in a guide tube (28) which can be fixed proximally in handle part (3).

12. Endoscope according to one of claims 1 to 11, characterised in that connection part (5) has a longitudinal groove (35) which corresponds to a positioning pin (36) on handle part (3).

13. Endoscope according to one of claims 1 to 12, characterised in that a round wire (37), which may engage in an annular groove (38) on connection part (5), is arranged on the distal end of handle part (3).

## Revendications

1. Endoscope (1) comportant une tige flexible (4), dont l'extrémité proximale se prolonge par une partie de raccordement (5) et dont l'extrémité distale peut être déviée dans différentes positions par déplacement d'un fil d'actionnement (6), et comportant une partie formant poignée (3), à l'extrémité distale de laquelle peut être accouplée de façon amovible la partie de raccordement (5) et à l'extrémité proximale de laquelle est disposée une partie formant occulaire (2), une unité de commande (7) devant être actionnée manuellement, étant prévue, pour le fil d'actionnement (6), dans la partie formant poignée (3), caractérisé en ce que l'extrémité proximale (19) du fil d'actionnement (6) est reliée à un logement (13,14c) situé dans l'unité de commande (7) et que le logement (13,14c) doit être ouvert moyennant l'actionnement d'un levier de déplacement (8) pour libérer l'extrémité mentionnée (19) du fil.

2. Endoscope selon la revendication 1, caractérisé en ce que le levier de déplacement (8) est déplaçable dans un sens à partir d'une position neutre pour ouvrir le logement (13,14c) et que le logement (13,14c) est fermé dans la position neutre et lors du déplacement du fil d'actionnement (6) et est relié, selon une liaison par formes complémentaires, à l'extrémité proximale (19) du fil.

3. Endoscope selon l'une des revendications 1 et 2, caractérisé par le fait que le levier de déplacement (8) et une partie intérieure (8a) du levier sont reliés entre eux rigidement et qu'une cornière de fixation (10) peut pivoter autour d'un axe de support (15) à l'encontre de la force d'un ressort (12), qui fait partie de l'unité de commande (7).

4. Endoscope selon l'une des revendications 1 à 3, caractérisé en ce qu'à partir de la position neutre, lors d'un déplacement du levier de déplacement (8) dans la direction distale, une tige de butée (11), qui est située à l'intérieur de l'unité de commande (7), agit sur une branche (14a) de la cornière de fixation (10) de telle sorte que le ressort (12), qui fait partie de l'unité de commande, est comprimé et que le logement (13,14c) s'ouvre.

5. Endoscope selon l'une des revendications 1 à 4, caractérisé en ce que le fil d'actionnement (6) prend appui sur le côté distal d'une partie plus épaisse (6a) du fil, au moyen d'un ressort de pression (17), contre une butée (18) de la partie de raccordement (5), de manière à être déplaçable longitudinalement.

6. Endoscope selon la revendication 5, caractérisé en ce que le fil d'actionnement (6) comporte, sur le côté proximal, une partie (19) pourvue d'un filetage et qui peut engrener, selon une liaison par formes complémentaires, avec une demi-coque filetée (13), sous l'action de la force d'un ressort.

7. Endoscope selon la revendication 6, caractérisé en ce qu'avant une contrainte excessive du fil d'actionnement (6), la liaison par formes complémentaires existant entre la partie filetée (19) et la demi-coque filetée (13) peut être supprimée à l'encontre de la force du ressort (12) qui fait partie de l'unité de commande.

8. Endoscope selon l'une des revendications 1 à 7, caractérisé en ce que le câble (22) de transmission d'image de l'endoscope (1) est guidé, approximativement à partir du milieu de la partie de raccordement (5), dans un tube (27).

9. Endoscope selon l'une des revendications 1 à 8, caractérisé en ce que la partie formant poignée (3) possède un tube de guidage (27a), qui est agencé de manière à loger le câble (22) de transmission d'image, conjointement avec son tube (27), et se termine directement devant une butée (33) de la partie formant poignée (3).

10. Endoscope selon la revendication 9, caractérisé en ce qu'une plaquette distale de fermeture (31) de la partie formant occulaire (2) s'applique contre la face proximale de la butée (33).

11. Endoscope selon l'une des revendications 1 à 10, caractérisé en ce que le fil d'actionnement (6) est guidé dans un tube de guidage (28), qui peut être fixé de façon proximale dans la partie formant poignée (3).

12. Endoscope selon l'une des revendications 1 à 11, caractérisé en ce que la partie de raccordement (5) possède une rainure longitudinale (35), qui correspond à une tige de positionnement (36) de la partie formant poignée (3).

13. Endoscope selon l'une des revendications 1 à 12, caractérisé en ce que sur l'extrémité distale de la partie formant poignée (3) est disposé un fil cylindrique (37), qui peut s'encliqueter dans une rainure annulaire (38) de la partie de raccordement (5).
